# EUROPEAN PATENT APPLICATION

(11) **EP 1 721 989 A1**
(43) Date of publication of application: **15.11.2006**
(21) Application number: 05708079.8
(22) Date of filing: 10.02.2005
(51) Int. Cl.: C12P 7/66, C12N 15/53

(54) **METHOD OF PREPARING MENADIONE WITH A RECOMBINANT MICRO-ORGANISM**

(30) Priority: 11.02.2004 ES 200400425
(71) Applicant: Institut Univ De Ciència I Technologia, 08100 Mollet del Vallès (ES)
(72) Inventor: ESTEVEZ COMPANY, Carles, E-08100 Mollet del Vallès (ES); SOLDEVILA FABREGA, Andreu, E-08100 Mollet del Vallès (ES); CASTELLS BOLIART, Josep, E-08100 Mollet del Vallès (ES); MONTILLA AREVALO, Rafael, E-08100 Mollet del Vallès (ES)
(74) Representative: Barlocci, Anna
(86) International application number: PCT/ES2005/000065
(87) International publication number: WO 2005/078112

(57) **Abstract**

The invention provides an alternative process for the preparation of menadione, also known as vitamin K₃, lacking of acid waste and the use of toxic chemicals, which leads to a safe, economical and environmentally benign process. The process comprises cultivating bacteria Escherichia coli in a suitable culture medium comprising one or more substrates, preferably 2-methyl-1-naphtol, and recovering menadione from the culture medium; said bacteria having the ability to produce menadione because they comprise a recombinant DNA vector encoding a quinol oxidase. The use of microorganisms as biocatalysts allows a selective process in which no isomer of menadione is substantially produced.

SEQUENCE LISTING

<110>  INSTITUT UNIV DE CIÈNCIA I TECNOLOGIA

<120>  Preparation process of menadione with a recombinant microorganism

<130>  P763EP00

<140>  -
<141>  2006-09-07

<150>  PCT/ES2005/000065
<151>  2005-02-10

<150>  ES200400425
<151>  2004-02-11

<160>  5

<170>  PatentIn version 3.3

<210>  1
<211>  34
<212>  DNA
<213>  Artificial

<220>
<223>  PCR forward primer

<400>  1
caccgagaaa agattttgag gaagtatgca cttc                                    34


<210>  2
<211>  29
<212>  DNA
<213>  Artificial

<220>
<223>  PCR reverse primer

<400>  2
gtataattcg ttatggcctg aatgctctg                                          29


<210>  3
<211>  18
<212>  DNA
<213>  Artificial

<220>
<223>  Sequencing forward primer

<400>  3
ttcctcgacg ctaacctg                                                      18


<210>  4
<211>  20
<212>  DNA
<213>  Artificial

<220>

<223>  Sequencing reverse primer

<400>  4
tagttattgc tcaggggtgg                                                   20


<210>  5
<211>  4176
<212>  DNA
<213>  Escherichia coli

<400>  5
gatgacgatg acaagctggg aattgatccc ttcaccgaga aaagattttg aggaagtatg      60

cacttcagat ttattacatc aataggaagg atggtgatct tcttgttcag agcattaaaa     120

ccgttgcttg tattagcgct gttaactgtg gttttcgtcc ttgggggatg cagcaatgct     180

tcagtactag atccgaaagg acctgtagct gaacagcaaa gtgatttaat cctgttatct     240

atcggattta tgctgtttat cgtcggggtc gtctttgttc tatttaccat tattttagta     300

aaataccgcg accgtaaagg caaagataat ggatcttata acccagaaat tcacggtaac     360

acgtttttag aagtagtctg gacagtgatc ccaattttaa tcgtcattgc actttctgtg     420

ccaacagtac agacgatata ttcgttagaa aaagctcctg aagcaacaaa agacaaagag     480

cctcttgttg tttatgctac ttcggtagac tggaaatggg tatttagcta ccctgaacag     540

gatattgaaa cggtcaacta cttaaacatt cctgtcgacc gcccgatctt gttcaaaatc     600

tcatctgcag attcaatggc ttcgctatgg attcctcagc ttggagggca aaaatatgcg     660

atggcaggaa tgctgatgga tcaatactta caggctgaca aagtgggtac ttacgaaggc     720

cgcaacgcga acttcacagg tgaacatttt gcagaccaag agtttgatgt gaatgcagtc     780

acagaaaaag actttaacag ctgggtgaaa aagactcaga acgaggctcc taagctgaca     840

aaagagaagt atgatgaatt gatgcttcct gaaaatgtgg acgaattaac gttctcttct     900

actcacttga aatacgttga tcacggacaa gacgctgagt acgcgatgga ggcgcgcaaa     960

cgccttggct atcaagccgt ttcaccgcat tctaaaacag atccgtttga aaacgtaaag    1020

aaaaacgaat ttaagaagtc tgatgataca gaagaatgat attgatcagg aaaggaggaa    1080

gcctgcatga aattcaaatg ggacgaattt ttcgtaactg gtgacccatt aattctgggc    1140

gcacaagttt ccattgcgct ttcaaccatt gccattattt ttgttctgac ttacttcaaa    1200

aagtggaaat ggctgtggtc tgaatggata actactgttg accataaaaa actcggaatc    1260

atgtatatta tatctgctgt aattatgtta ttccgtggcg gtgtcgacgg tctgatgatg    1320

cgtgcccagc tagcgcttcc gaataacagt tttttagact caaaccacta taatgaaatt    1380

tttacaactc acggtacaat catgatcatc ttcatggcga tgccgttctt aatcggtctt    1440

atcaacgttg ttgtgcctct tcaaatcggt gcccgcgacg ttgcgttccc ttacttgaac    1500

aacctgagct tctggacgtt ctttgtaggg gcgatgcttt tcaatatttc tttcgttatc    1560

ggaggttctc cgaacgcagg ctggacgagt tatatgccgc ttgcaagtaa cgacatgagc 1620
cctgggccgg gtgagaacta ctacctcctc ggacttcaga ttgccggtat cggtaccctg 1680
atgacaggga tcaactttat ggtaacaatc ttaaaaatga gaacaaaagg catgacatta 1740
atgcgtatgc cgatgttcac atggacaaca ctgatcacaa tggttattat cgtgtttgcg 1800
ttccctgtac ttacagtagc attagcactt ttatcatttg atcgtttgtt cggcgctcac 1860
tttttcacat tggaagcagg cggtatgccg atgctttggg ctaacttgtt ctggatttgg 1920
ggacatcctg aagtatatat cgttattctt ccggcgtttg gtattttctc agaaatcatc 1980
tcaagctttg caagaaaaca attgtttggt tacaaagcga tggttggttc tattatcgcc 2040
atctctgtat tgagtttctt agtttggact caccacttct tcacaatggg gaacagtgcg 2100
tccgttaact cattcttctc aattacgaca atggcaatct caatcccaac cggggttaaa 2160
atctttaact ggctctttac aatgtataaa ggccgaatca gctttacaac acctatgctg 2220
tgggcgcttg cgttcattcc taacttcgtt atcggcgggg ttacaggggt tatgcttgcg 2280
atggcagcag cggattatca ataccataat acgtacttcc ttgtatctca cttccactat 2340
gtgctgattg cgggtactgt atttgcttgt ttcgccggat ttattttctg gtatccaaaa 2400
atgttcggcc acaagctgaa cgaaagaatc ggaaaatggt tcttctggat ctttatgatc 2460
gggttcaata tttgtttctt cccgcaatat ttcttggggc ttcaaggcat gccgcgccgt 2520
atttacacat acggaccgaa tgacggctgg acaacattga actttatctc aacagtcgga 2580
gctttcatga tgggtgtagg attcttaatc ctttgctata acatctacta cagcttccgt 2640
tactctactc gtgaaatcag cggagattca tggggtgtgg gacgtacgct tgattgggcg 2700
acttcttctg cgattccgcc gcattacaac tttgcggtgc ttcctgaagt caaatctcaa 2760
gatgcattcc tgcatatgaa agaagaaaaa actgaattat accctgaaag caaattcaag 2820
aaaatccata tgccaagcaa ctcaggcaga ccgttcttta tgtctgttgc cttcggtctc 2880
gcaggctttg gacttgtctt tgaatggtac tggatgggcg tagtcggatt gatcggagtc 2940
ctgctctgca tggttctgcg ttcattcgaa tacgacaacg gctactacat tagtgttgat 3000
gaaataaaag agacggaaag aaagatttcc gaataaggag gcgtgagtta tggaacatgc 3060
agaacacggc aattctaacg cgcctatgga atatcaatct gaaaccggca gacttaatat 3120
tctcgggttt tggatctttt taggggcaga aattgtgttg ttctcaacac tatttgcaac 3180
cttcttcgtt cttaaaaaca gaacagctgg aggtgtcttg ccagacgaac tatttgaagt 3240
caatctggta atgattatga cgttcttgct gctgatcagc agctttacct gcgggatcgc 3300
tgttcatgaa atgcgccgcg gaagtttaaa aggcgtagtc atttggacaa ttatcactct 3360
tcttctcggt gccggcttcg tcggatgcga gatcaacgag tttgttcact atgtacatga 3420

aggagcggct ctcagtacaa gtgcgttctg gtctggattc tttgttttac ttggaactca 3480

cggaactcac gtaacaatcg ggattttctg gatcacagga attctgattc agttgaagaa 3540

acgcggctta actccgcaaa cttcatctaa aatctttatc tcaagcttgt actggcactt 3600

cttagatgtt gtatggattt tcatctttac aggtgtctat ctcatgggat tggggggtct 3660

gtaaaatggc aaacaaatct gctgaacaca gtcactttcc atggaaacac attgtaggtt 3720

ttatcctgtc tatcgttttg accctgctgg ctctatgggt tgcagtgtat actgacctga 3780

gctcatcagc aaaactttgg attatcttcg gcttcgcctt cattcaggcg gcgctgcagc 3840

ttctcatgtt catgcatatg acagagagcg aaaacggcac gatccaagtc ggaaacacgc 3900

tgttcggatt ctttggcgcg atcgttatcg tacttggatc catctggatt ttcgcggctc 3960

actatcacca tggtgaccat atggacggaa atcctcctgg aggcgctgaa cactcagagc 4020

attcaggcca taacgaatta tacaagggcg agctcaagct tgaaggtaag cctatcccta 4080

accctctcct cggtctcgat tctacgcgta ccggtcatca tcaccatcac cattgagttt 4140

gatccggctg ctaacaaagc ccgaaaggaa gctgag 4176

## Description

This invention relates to the field of industrial microbiology, and specifically to a new preparation process of a low molecular weight compound using microorganisms transformed with recombinant DNA.

### BACKGROUND ART

Menadione or 2-methyl-1,4-naphtalenedione is also known as vitamin K₃ or synthetic K₃ vitamin and it has the accompanying chemical formula (I). Menadione plays an essential role in blood coagulation, acting in the synthesis of protrombine and blood coagulation factors VII, IX and X. It also acts in the conversion of hepatic glucose into glycogen. Apparently, it has an important role in the synthesis of human bones, reason for which it is also used for the prevention of osteoporosis. The necessary menadione daily income ranges from 5 µg (children under 3 years old) to 80 µg (adults), which is usually covered by a diet rich in vegetables. Deficiency in menadione, mainly due to intestinal alterations, may lead to coagulation and vascular problems. For these reasons, menadione is considered an active agent produced and used worldwide, both in animal and human therapy.

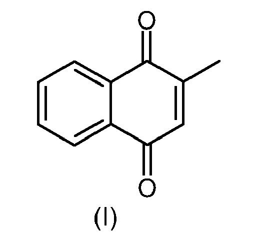

At industrial level menadione is mainly synthesized by an extremely pollutant oxidative process, which includes very dangerous and toxic chemical substances. This process involves the oxidation of 2-methylnaphtalene (II-4) with chromic acid or derivatives (CrO₃ or Na₂Cr₂O₇, cf. US 2.402.226). Numerous processes for the manufacture of menadione have been suggested trying to improve the current industrial synthesis. One of them is based on the oxidation with hydrogen peroxide (cf. US 2.373.003), but it has been proved not feasible at industrial level. Another process is based on the oxidation of 2-methylnaphtalene with sodium dichromate, sulfuric acid, acetic acid and pyridine (cf. US 3.751.437), which also are highly contaminant chemicals.

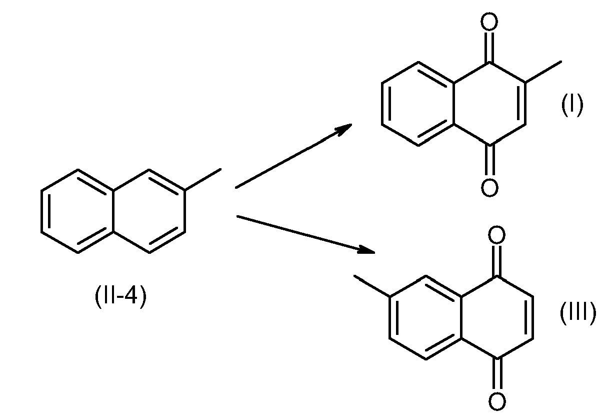

One of the main problems of synthetic pathways known in the art is the concomitant production of a regioisomer (III) which does not have the pharmacological activity of menadione. The presence of this subproduct makes more difficult and expensive the steps of purification of menadione. There have been several other attempts trying to improve the above-mentioned pollutant processes, but their scale-up to industrial level have not been successful. Examples of these alternative processes involve the oxidation in a multitubular reactor with vanadium oxide, potassium pyrosulfate and potassium sulfate, or the oxidation by potassium hydrogensulfate and a manganese-containing porphyrine.

In order to solve the pollution problems of chemical preparation processes, a bio-based approach has been reported which involves the use of a bacterium from the genus Rhodococcus (cf. JP 6022775), although it has not been fully industrialized yet. Thus, it seems highly desirable to provide alternative industrial microbiological processes for the preparation of menadione, which are both non-pollutant and regioselective.

### SUMMARY OF THE INVENTION

The inventors have surprisingly found that the enzyme quinol oxidase shows a high activity in presence of 2-methyl-1,4-naphtalenediol or menadiol, resulting in the production of menadione. Quinol oxidase, named simply "qox" in this description, functions as a terminal oxidase in the respiratory chain of some bacteria, reducing O₂ to water. Terminal oxidases have different names depending on the characteristics of the enzyme and on the microorganism. The inventors have found that the Bacillus subtilis quinol oxidase, also known as quinol oxidase aa3-600 or cytochrome aa3 quinol oxidase, is particularly suitable in this invention.

The inventors have taken advantage of the potential of the enzyme qox to develop an alternative process for the preparation of menadione. Qox in its natural environment does not allow the obtention of a high yield of menadione as is desired at industrial level. To overcome this serious limitation, the inventors have used recombinant DNA technology to design an expression vector comprising quinol oxidase and appropriate elements to overexpress quinol oxidase in the selected host bacterium. The designed expression vector also facilitates the solubilization and the purification of quinol oxidase. The invention process for the preparation of menadione involves the elimination of acid waste and toxic chemicals, so it is economical, safe and environmentally benign. An additional advantage is that it is a selective process in which no isomer of menadione is substantially produced.

The invention provides a recombinant DNA expression vector for the production of menadione in Escherichia coli, said vector comprising a nucleotide sequence encoding a quinol oxidase and nucleotide sequences which allow said vector to be selectable and autonomously replicable in Escherichia coli. In this description "Escherichia coli qox" means Escherichia coli transformed with the recombinant expression vector which comprises quinol oxidase nucleotide sequence. The recombinant DNA vector allows Escherichia coli to produce quinol oxidase and, when medium conditions are suitable, quinol oxidase catalyzes the obtention of menadione. In a particular embodiment of the invention, the quinol oxidase gene introduced in the expression vector is the cytochrome aa3 quinol oxidase gene of Bacillus subtilis bacteria. Particularly, the Bacillus subtilis bacteria come from the Bacillus subtilis DSMZ 402 bacterial strain and, more particularly, the nucleotide sequence encoding the cytochrome aa3 quinol oxidase of Bacillus subtilis DSMZ 402 comprises SEQ ID NO: 5.

The construction of the recombinant DNA expression vector is carried out with conventional recombinant DNA technologies, i.e. procedures to join together DNA segments in a cell-free system. The term "vector" refers to a DNA molecule originating from a virus, a plasmid, or a cell of a higher organism, into which another DNA fragment of appropriate size can be integrated (cloned) without loss of the vector capacity for self-replication. Examples are plasmids, cosmids and yeast artificial chromosomes. Vectors are often recombinant molecules containing DNA sequences from several sources. The term "expression vector" means a vector that further contains the necessary regulatory sequences for transcription and translation of the cloned gene or genes. Circular or linearized DNA vectors are useful for this invention.

To allow the vector of the invention to be selectable and autonomously replicable in Escherichia coli, the selected vector must be compatible with the selected Escherichia coli host cells. In a preferred embodiment, the nucleotide sequence which allow said vector to be selectable and autonomously replicable in Escherichia coli is the T7 promoter-encoding gene which permits the T7 RNA polymerase of the selected strain of Escherichia coli to bind to the promoter. The term "selectable" means that the vector remains stable in the descendant bacteria. The selection is achieved by stringent medium conditions according to the introduction of an appropriate selectable marker gene in the vector whose expression allows to identify cells that have been successfully transformed with the vector. The selectable marker gene is often an antibiotic-resistant gene. Preferred selectable marker genes in this invention give resistance to kanamycin, tetracycline, carbenicillin and more preferably, to ampicillin.

In another particular embodiment of the invention, the expression vector further comprises nucleotide sequences which allow to increase the solubility and/or to improve the purification of the resulting quinol oxidase. To this end nucleotide sequences encoding a polyhistidine tag at C-terminal position of the quinol oxidase, a thioredoxin tag at N-terminal position of the quinol oxidase or gluthatione S-transferase fusion proteins are useful.

The invention also provides Escherichia coli bacteria transformed with the recombinant DNA expression vector described above, or descendant bacteria thereof. A person skilled in the art will appropriately choose the expression system, constituted by an initial vector and an Escherichia coli bacterial strain to maximize the production of menadione. In a particular embodiment, the Escherichia coli belongs to BL21 bacterial strain. Suitable expression vectors for Escherichia coli BL21 are, for instance, pET vectors, trcHis vectors and pUB vectors (all of them from Invitrogen), and pGEX vectors and GST vectors (from Amersham). Escherichia coli DH5 alfa bacterial strain in combination with pUC vectors and Escherichia coli F' in combination with PSL vectors, PEZZ vectors or M13 vectors (all of them from Amersham) are also useful in this invention.

The invention also provides a preparation process of menadione comprising the steps of: (i) cultivating bacteria Escherichia coli in a suitable culture medium comprising one or more substrates of formula (II), wherein X and Y are radicals independently selected from H and OH, and (ii) recovering menadione from the culture medium; said bacteria having the ability to produce menadione because they comprise a recombinant DNA vector encoding a quinol oxidase. In this description, the term "the ability to produce menadione" means that the bacterium accumulates menadione in the medium in such amount that can be collected. Possible substrates are II-1, II-2, II-3 and II-4. In a preferred embodiment, the substrate used is 2-methyl-1-naphtol (II-1).

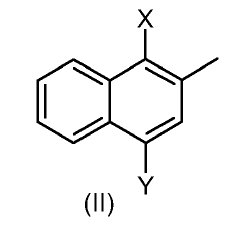

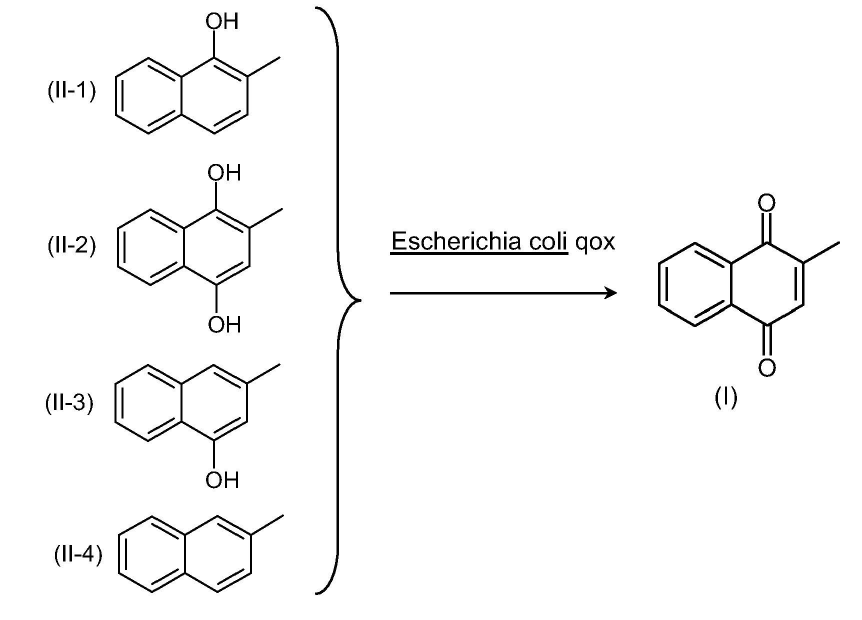

In a particular embodiment of the invention, the bacterial cells are disrupted or whole. When cells are disrupted quinol oxidase can be purified. In a preferred embodiment, the bacterial cells have been precultivated in a culture medium comprising tryptone, yeast extract, sodium chloride and an antibiotic selected from the group consisting of kanamycin, tetracycline, carbenicillin and ampicillin. In other embodiments of the invention, the culture medium used for preparing menadione comprises a linear saturated hydrocarbon of 10 to 18 carbon atoms, particularly hexadecane, in a concentration in the culture medium of 10% v/v.

Throughout the description and claims the word "comprise" and its variations are not intended to exclude other technical features, additives, components, or steps. The abstract of this application is incorporated herein as reference. Additional objects, advantages and features of the invention will become apparent to those skilled in the art upon examination of the description or may be learned by practice of the invention. The following particular embodiments, drawings and sequences listing are provided by way of illustration, and are not intended to be limiting of the present invention.

### BRIEF DESCRIPTION OF THE DRAWINGS

FIG. 1 shows a genetic map of the initial expression vector pET102/D-TOPO® before cloning.
FIG. 2 shows a genetic map of the resulting recombinant expression vector.

### DETAILED DESCRIPTION OF PARTICULAR EMBODIMENTS

### Detailed description of the drawings

FIG. 1 shows a genetic map of the initial expression vector pET102/D-TOPO® of 6315 nucleotides. T7 promoter: bases 209-225; T7 promoter priming site: bases 209-228; lac operator (lacO): bases 228-252; ribosome binding site (RBS): bases 282-288; His-patch (HP) thioredoxin ORF: bases 298-627; "TrxFus forward" priming site: bases 607-624; EK recognition site: bases 643-657; TOPO® recognition site 1: bases 670-674; overhang: bases 675-678; TOPO® recognition site 2: bases 679-683; V5 epitope: bases 700-741; polyhistidine (6xHis) region: bases 751-768; "T7 reverse" priming site: bases 822-841; T7 transcription termination region: bases 783-911; bla promoter: bases 1407-1505; Ampicillin (bla) resistance gene (ORF): bases 1506-2366; pBR322 origin: bases 2511-3184; ROP ORF: bases 3552-3743 (complementary strand); lacl ORF: bases 5055-6146 (complementary strand).

FIG. 2 shows a genetic map of the resulting recombinant expression vector after cloning qox nucleotide sequence. The vector elements are the same that in FIG. 1 but with bases moved. Qox insertion point is at "overhang", bases 675-678. The cloned sequence read by sequencing corresponds to SEQ ID NO: 5 with 4177 bases (it includes bases until "T7 reverse priming site"), which is not exactly the whole nucleotide sequence cloned into the vector because of inherent reasons in sequencing.

### Bacterial strain and expression vector used

Escherichia coli BL21 (pET102 Directional TOPO® Expression Kit, Invitrogen) is a commercial strain which is used for regulated expression of heterologous genes. It contains the gene for the T7 RNA polymerase which makes the strain compatible with the use of pET vectors containing T7 promoter for the overexpression of recombinant proteins induced with isopropyl-β-D-thiogalactoside (IPTG).

pET102/D-TOPO® vector (pET102 Directional TOPO® Expression Kit, Invitrogen) contains a pBR322ori for plasmid replication, the ampicillin resistance gene, the T7 promoter which permits binding of T7 RNA polymerase, the lac operator which permits inhibition of expression when IPTG is not present, a ribosome binding site for translation of RNA, a His-patch-thioredoxin for the increase of solubility of the fusion protein and a polyhistidine tag (6xHis) for detection and purification of the fusion protein.

### Construction of the Escherichia coli qox

The commercial strain Escherichia coli BL21 was genetically modified by a transformation assay with the plasmid pET102/D-TOPO® containing the quinol oxidase (qox) gene of Bacillus subtilis DSMZ 402 (accession number for the deposit in the "Deutsche Sammlung von Mikroorganismen und Zellkulturen GmbH"; the strain was purchased directly to the DSMZ collection).

Obtention of qox gene: Qox, also known as quinol oxidase aa3-600 or cytochrome aa3 quinol oxidase, is the main oxidase in the exponential growth phase of Bacillus subtilis. It consists of 4 subunits (qoxA, B, C and D) and the length of its DNA sequence is 3.9 Kb (GenBank, protein Accession Numbers P34959 (qoxD), P34958 (qoxC), P34956 (qoxB) and P34957 (qoxA); gene Accession Number M86548).

Qox gene was amplified by PCR using 2 units of Platinum Pfx enzyme (Invitrogen), 1mM of Mg₂SO₄ and 1x amplification buffer, 200 µM of dNTPs and 0.3 µM of each primer. Primers used in the PCR were "qox forward" 5' CACCGAGAAAAGATTTTGAGGAAGTATGCACTTC 3' (SEQ ID NO: 1; 79 °C of melting temperature) and "qox reverse" 5' GTATAATTCGTTATGGCCTGAATGCTCTG 3' (SEQ ID NO: 2; 75 °C of melting temperature). Primers were designed from qox gene sequence reported in GenBank, Accession Number M86548. The PCR reaction was performed with an initial denaturation step at 94 °C for 3 min followed by 36 temperature cycles consisting of a denaturation step at 94 °C for 1 min, an annealing/extension step at 60 °C for 1.5 min and a step at 68 °C for 4 min. After the 36 cycles, the sample was subjected to 68 °C for 10 min and finally at 4 °C indefinitely. PCR product was analyzed by agarose gel electrophoresis, and the DNA band was purified from the gel (S.N.A.P.^{™} UV-Free Gel Purification Kit, Invitrogen).

Cloning into pET102/D-TOPO® vector and cells transformation: The DNA fragment was cloned into pET102/D-TOPO® vector and Escherichia coli BL21 bacteria were transformed with the vector by thermal shock. Transformed cells were analyzed by DNA restriction with 10 units of the enzyme HindIII. The positive clone was sequenced using the sequencing primers "TrxFus forward" 5' TTCCTCGACGCTAACCTG 3' (SEQ ID NO: 3) and "T7 reverse" 5' TAGTTATTGCTCAGGGGTGG 3' (SEQ ID NO: 4). The DNA sequence 5'->3' of the resulting fusion gene is SEQ ID NO: 5. This sequence SEQ ID NO: 5 does not exactly correspond to the whole nucleotide sequence cloned into pET102/D-TOPO® vector because SEQ ID NO: 5 is the result of sequencing. Inherently in sequencing, primers for sequencing does not allow to read the first and the end 30-40 nucleotides of the complete cloned sequence.

### Assay 1: Preparation of menadione with whole cells of Escherichia coli qox

Preliminary growth of the microorganism: Precultures of Escherichia coli qox were grown at pH 7 in trypticase soy solid medium supplemented with ampicillin. One colony of the culture was passed into nutrient broth n° 2 (Oxoid) containing Lab-lemco powder (10 g/l), peptone (10 g/l) and NaCl (5g/l), supplemented with 200 mg/l of ampicillin. It was incubated at 37 °C with vigorous shaking (200 rpm), adding 50-200 mg/l of IPTG after three-four hours. Qox was overexpressed for eight hours, followed by harvesting cells with a centrifugation step of 20 min at 4500 rpm, and washing them in 50 mM of phosphate buffer.

Menadione preparation: Cells were passed into 50 mM of phosphate buffer supplemented with 25 mg/I of 2-methyl-1-naphtol using DMSO as solvent. Incubation was carried out at 37 °C and shaking at 200 rpm for a reaction time of two, five and ten days. After those periods, cells were harvested and menadione was extracted from the broth using dichloromethane. The product was concentrated and analyzed by gas chromatography-mass spectrometry (GC-MS). Results are shown in TABLE 1 (Assay 1).

### Assay 2: Preparation of menadione with whole cells of Escherichia coli gox and addition of hexadecane

Preliminary growth of the microorganism: This step was carried out as in Assay 1.

Menadione preparation: Cells were passed into 50 mM of phosphate buffer supplemented with 25 mg/l of 2-methyl-1-naphtol using hexadecane as solvent in a proportion of 10% (v/v). Incubation was carried out at 37 °C and shaking at 200 rpm for a reaction time of two, five and ten days. After those periods, hexadecane was collected by decanting, menadione was extracted with a solid phase extraction system and was eluted in methanol. Menadione was concentrated and analyzed by GC-MS. Cells were harvested and residual menadione was completely extracted from the broth using dichloromethane. The product was concentrated and analyzed by GC-MS. Results are shown in TABLE 1 (Assay 2) and the distribution into hexadecane was of >99%.

### Assay 3: Preparation of menadione with completely or Partly purified quinol oxidase of Escherichia coli qox

Preliminary growth of the microorganism: This step was carried out as in Assay 1.

Cell disruption and quinol oxidase purification: For a partial purification of the protein, cells were disrupted by the addition of 160 mg of lisozyme and one hour of incubation at 0 °C followed by three freezing-thawing cycles (-80 °C / 37 °C) and a final step for reducing viscosity adding 1000 units of deoxyribonuclease I. To achieve the complete purification of quinol oxidase, the previous mixture was passed throughout an affinity chromatography column.

Menadione preparation: In two different assays, the mixture of disrupted cells and purified quinol oxidase fractions were passed into 50 mM of phosphate buffer supplemented with 25 mg/l of 2-methyl-1-naphtol using DMSO as solvent. Incubation was carried out at 37 °C and shaking at 200 rpm for a reaction time of two, five and ten days. After those periods, cells were harvested and menadione was extracted from the broth using dichloromethane. The product was concentrated and analyzed by GC-MS. The results for both assays (with the mixture of disrupted cells and with purified quinol oxidase fractions) are shown in TABLE 1 (Assay 3).

### Assay 4: Preparation of menadione with completely or partly purified quinol oxidase of Escherichia coli qox and addition of hexadecane

Preliminary growth of the microorganism: This step was carried out as in Assay 1.

Cells disruption and quinol oxidase purification: This step was carried out as in Assay 3.

Menadione preparation: In two different assays, the mixture of disrupted cells and purified quinol oxidase fractions were passed into 50 mM of phosphate buffer supplemented with 25 mg/l of 2-methyl-1-naphtol using hexadecane as solvent in a proportion of 10% (v/v). Incubation was carried out at 37 °C and shaking at 200 rpm for a reaction time of two, five and ten days. After those periods, cells were harvested and menadione was extracted from the broth using dichloromethane. The product was concentrated and analyzed by GC-MS. The results for both assays (with the mixture of disrupted cells and with purified quinol oxidase fractions) are shown in TABLE 1 (Assay 4).

**TABLE 1. Results of menadione preparation assays with Escherichia coli qox**

| assay | Escherichia coli qox treatment | hexadecane | 2-methyl-1-naphtol (mg/l) | production in 2 days (mg/l) | production in 5 days (mg/l) | production in 10 days (mg/l) |
|---|---|---|---|---|---|---|
| 1 | whole cells | no | 25 | 16.5 | 25 | 25 |
| 2 | whole cells | yes | 25 | 15.8 | 25 | 25 |
| 3 | mixture of disrupted cells | no | 25 | 6 | 25 | 25 |
| | purified qox fractions | no | 25 | 6 | 25 | 25 |
| 4 | mixture of disrupted cells | yes | 25 | 8 | 25 | 25 |
| | purified qox fractions | yes | 25 | 8 | 25 | 25 |

## Claims

1. A recombinant DNA expression vector for the production of menadione in Escherichia coli, said vector comprising a nucleotide sequence encoding a quinol oxidase and nucleotide sequences which allow said vector to be selectable and autonomously replicable in Escherichia coli.

2. The recombinant DNA expression vector according to claim 1, which further comprises nucleotide sequences which allow to increase the solubility and/or to improve the purification of the quinol oxidase.

3. The recombinant DNA expression vector according to claim 2, wherein one of the nucleotide sequences encodes a polyhistidine tag at C-terminal position of the quinol oxidase.

4. The recombinant DNA expression vector according to claim 2, wherein one of the nucleotide sequences encodes a thioredoxin tag at N-terminal position of the quinol oxidase.

5. The recombinant DNA expression vector according to any of the claims 1-4, wherein the nucleotide sequence which allow said vector to be selectable and autonomously replicable in Escherichia coli is the T7 promoter-encoding gene which permits the T7 RNA polymerase of the selected strain of Escherichia coli to bind to the promoter.

6. The recombinant DNA expression vector according to any of the claims 1-5, wherein the quinol oxidase is the cytochrome aa3 quinol oxidase of Bacillus subtilis bacteria.

7. The recombinant DNA expression vector according to claim 6, wherein the Bacillus subtilis bacteria come from the Bacillus subtilis DSMZ 402 bacterial strain.

8. The recombinant DNA expression vector according to claim 7, wherein the nucleotide sequence encoding the cytochrome aa3 quinol oxidase of Bacillus subtilis DSMZ 402 comprises SEQ ID NO: 5.

9. Escherichia coli bacteria transformed with the recombinant DNA expression vector according to any of the claims 1-8, or descendant bacteria thereof.

10. Escherichia coli BL21 strain bacteria transformed with the recombinant DNA expression vector according to any of the claims 1-8, or descendant bacteria thereof.

11. A preparation process of menadione (I) comprising the steps of: (i) cultivating bacteria Escherichia coli in a suitable culture medium comprising one or more substrates of formula (II), wherein X and Y are radicals independently selected from H and OH, and (ii) recovering menadione from the culture medium; said bacteria having the ability to produce menadione because they comprise a recombinant DNA vector encoding a quinol oxidase.

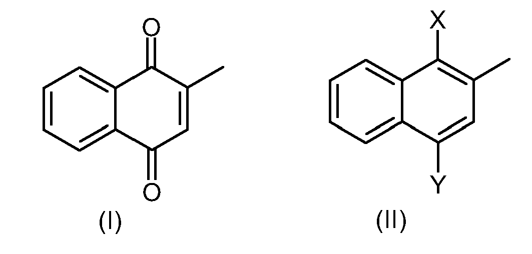

12. A preparation process of menadione (I) comprising the steps of: (i) cultivating the Escherichia coli bacteria according to any of the claims 9-10, in a suitable culture medium comprising one or more substrates of formula (II), wherein X is a radical selected from H and OH, and (ii) recovering menadione from the culture medium.

13. The process according to claim 12, wherein the bacterial cells are disrupted; optionally enriching the culture medium in quinol oxidase by separation of cellular waste.

14. The process according to claim 12, wherein the bacterial cells are whole.

15. The process according to any of the claims 12-14, wherein the culture medium comprises 2-methyl-1-naphtol.

16. The process according to any of the claims 12-15, wherein the bacterial cells have been precultivated in a culture medium comprising tryptone, yeast extract, sodium chloride and an antibiotic selected from the group consisting of kanamycin, tetracycline, carbenicillin and ampicillin.

17. The process according to any of the claims 12-16, wherein the culture medium comprises a linear saturated hydrocarbon of 10 to 18 carbon atoms.

18. The process according to claim 17, wherein the hydrocarbon is hexadecane.

19. The process according to claim 18, wherein the concentration of hexadecane in the culture medium is 10% v/v.
